# FASCICULE DE BREVET EUROPEEN

(11) **EP 0 729 936 B1**
(45) Date de publication et mention de la délivrance du brevet: **07.07.1999**
(21) Numéro de dépôt: 96400451.9
(22) Date de dépôt: 01.03.1996
(51) Int. Cl.: C07C 57/03, A61K 31/195

(54) **Procédé de synthèse d'acides acryliques alpha-substitués et N-(mercaptoacyl) amino acides**
Verfahren zur Synthese der alpha-substiuierten Akrylsäuren und N-(mercaptoacyl)aminsäuren
Process for the synthesis of alpha substituted acrylic acids and N-(mercaptoacyl)amino acids

(30) Priorité: 03.03.1995 FR 9502494
(43) Date de publication de la demande: 04.09.1996
(73) Titulaire: SOCIETE CIVILE BIOPROJET, F-75003 Paris (FR)
(72) Inventeur: Duhamel, Pierre, 76130 Mont Saint Aignan (FR); Duhamel, Lucette, 76130 Mont Saint Aignan (FR); Danvy, Denis, 76190 Yvetot (FR); Monteil, Thierry, 76130 Mont Saint Aignan (FR); Lecomte, Jeanne-Marie, 75003 Paris (FR); Schwartz, Jean-Charles, 75014 Paris (FR)
(74) Mandataire: Bernasconi, Jean

(56) Documents cités:
- EP-A- 0 419 327
- EP-A- 0 539 848
- DE-A- 2 853 732
- DE-C- 94 132
- FR-A- 1 555 338

## Description

La présente invention a trait à un procédé de synthèse d'acides acryliques α-substitués et à leur application à la synthèse de dérivés N-(mercaptoacyl) amino-acides.

Elle concerne plus particulièrement un nouveau procédé de synthèse d'acides acryliques α-substitués de formule générale (I) 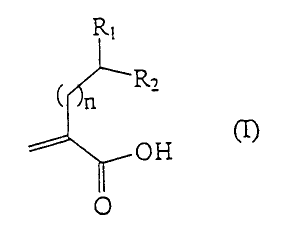 dans laquelle
R₁ représente un atome d'hydrogène ; un groupe alkyle ; un groupe cycloalkyle ; un groupe phényle éventuellement mono- ou polysubstitué par un atome d'halogène, un groupe trifluorométhyle, un groupe nitro, un groupe cyano, un groupe amino, un groupe diméthylamino, un groupe hydroxy, un groupe alcoxy-inférieur, un groupe phénoxy, un groupe benzyloxy, un groupe méthylthio, un groupe phényle, un groupe alkyle inférieur, un groupe phénylalkylène inférieur ; un groupe alpha et bêta naphtyle ; un groupement 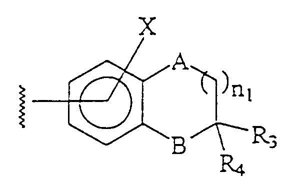 où
A est un groupe méthylène, un atome d'oxygène, un atome de soufre ou un atome d'azote,
B représente un des groupes ou atomes précités pour la définition de A,
n₁ est égal à 0 ou 1,
X représente un atome d'hydrogène, un atome d'halogène, un groupe hydroxy, un groupe alcoxy inférieur ou un groupe trifluorométhyle,
R₃ représente un atome d'hydrogène, un groupe phényle, un groupe alkyle inférieur, un atome d'halogène ou un groupe trifluorométhyle,
R₄ représente également un atome d'hydrogène ou l'un des groupes précités pour la définition de R₃,
R₂ représente un atome d'hydrogène ou l'un des groupes précités pour la définition de R₁ et
n varie de 0 à 10.

Les dérivés de formule (I) obtenus selon le procédé de l'invention sont, plus particulièrement, utiles dans la synthèse de N-(mercaptoacyl)amino-acides de formule (II). 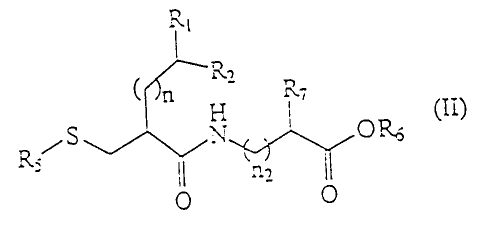 dans laquelle
R₁ et R₂ ont la même signification que dans la formule (I) ;
R₅ représente un atome d'hydrogène, un radical acyle aliphatique linéaire ou ramifié ou un radical acyle aromatique ;
R₆ représente un atome d'hydrogène, un radical alkyle inférieur, un radical phényle ou un groupe phénylalkylène inférieur ;
R₇ représente un atome d'hydrogène ; un groupe alkyle inférieur ; un groupe hydroxyalkylène inférieur ; un groupe phényle ; un groupe phénylalkylène inférieur ; un groupe hydroxyphénylalkylène inférieur ; un groupe aminoalkylène inférieur ; un groupe guanidinoalkylène inférieur ; un groupe mercaptoalkylène inférieur ; un groupe alkyle inférieur thioalkylène inférieur ; un groupe imidazolylalkylène inférieur ; un groupe indolylalkylène inférieur ; un groupe carbamylalkylène inférieur ; un groupe carboxyalkylène inférieur ;
n et n₂ varient de 0 à 10.

Par groupe alkyle inférieur, on entend des groupes alkyles à chaîne linéaire ou ramifiée, contenant de 1 à 6 atomes de carbone et, de préférence, 1 à 4 atomes de carbone.

Par groupe alkylène inférieur, on entend des groupes alkylènes contenant de 1 à 6 atomes de carbone et, de préférence, 1 à 4 atomes de carbone.

Par groupe alcoxy inférieur, on entend un groupe alcoxy contenant une chaîne linéaire ou ramifiée de 1 à 6 atomes de carbone.

Par groupe alkyle, on entend des groupes alkyles à chaîne linéaire ou ramifiée, contenant 1 à 20 atomes de carbone.

Par groupe cycloalkyle, on entend un cycle saturé de 3 à 7 atomes de carbone.

Les composés de formule (II) préférés sont les composés répondant aux formules suivantes :
1) 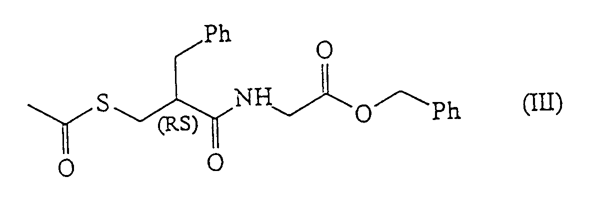 ou le N-(RS)-[2-acétylthiométhyl-1-oxo-3-phénylpropyl]-glycinate de benzyle ;
2) 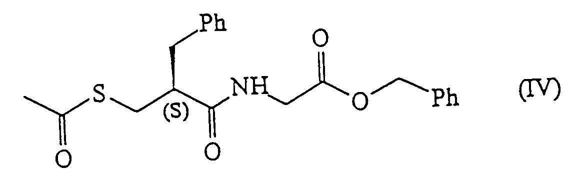 ou le N-(S)-[2-acétylthiométhyl-1-oxo-3-phénylpropyl]-glycinate de benzyle ;
3) 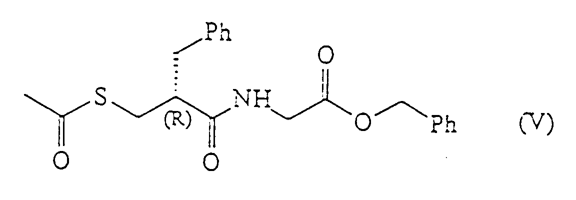 ou le N-(R)-[2-acétylthiométhyl-1-oxo-3-phénylpropyl]-glycinate de benzyle ;
4) 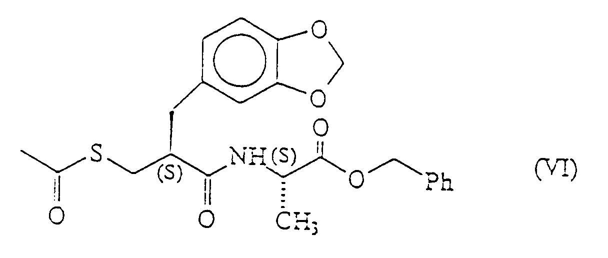 ou le N-(S)-[2-acétylthiométhyl-1-oxo-3-(3,4-méthylènedioxyphényl)propyl]-(S)-alaninate de benzyle.
5) 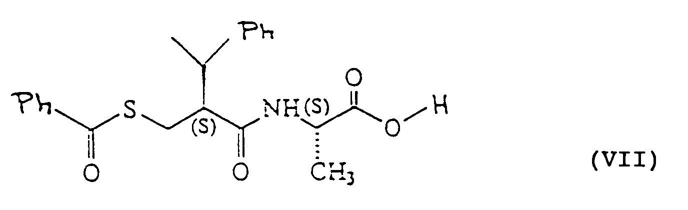 ou le N-[(2S, 3R)-2-benzoylthiométhyl-1-oxo-3-phénylbutyl)-(S)-alanine.

Les composés de formule (II) possèdent des propriétés pharmacologiques intéressantes. Ils exercent notamment une activité inhibitrice sur certaines enzymes, comme l'endopeptidase neutre (EC 3.4.24.11) et l'enzyme de conversion de l'angiotensine (EC 3.4.15.1). L'administration des composés de formule (II) permet donc de réduire ou de supprimer l'activité de ces enzymes, responsables respectivement de l'inactivation des enképhalines, du facteur atrial natriurétique, et de la transformation de l'angiotensine I en angiotensine II. En thérapeutique, ces composés exercent des activités antisécrétoires intestinales ou antihypertensives et sont utilisés dans le traitement de l'insuffisance cardiaque chronique. De plus, de tels composés peuvent être également utilisés dans le traitement de l'ostéoporose (PCT Int. Appl. WO. 94/21,242).

Les composés de formule (II) et, plus particulièrement le composé de formule (III), leur préparation et leur utilisation en thérapeutique ont été décrits dans le brevet européen n° 038 758.

Les composés de formule (II) et, plus particulièrement les composés de formules (IV) et (V), leur préparation et leur utilisation en thérapeutique ont été décrits dans le brevet français n° 2.623.498.

Les composés de formule (II) et, plus particulièrement le composé de formule (VI), leur préparation et leur utilisation en thérapeutique ont été décrits dans le brevet européen n° 0 419 327.

Peu de procédés de synthèse industrielle spécifiques des dérivés de formule (I) ont été décrits. On connaît le brevet européen n° 0 419 327 et la demande de brevet européen n° 0 539 848, faisant intervenir une réaction de Mannich sur un monoacide malonique. Toutefois, cette voie ne conduit pas directement aux acides acryliques de formule (I), mais aux esters correspondants, et ceci avec des rendements moyens. On connaît également la demande de brevet européen n° 0 539 848 faisant intervenir une réaction de Wittig entre du formol et l'anion d'un phosphono-ester alkylé pour accéder aux esters acryliques. Toutefois, cette voie nécessite la présence d'une base forte, telle que l'hydrure de sodium, et les rendements sont moyens.

La demanderesse a présentement découvert un procédé de synthèse industrielle d'acides acryliques α-substitués de formule (I) particulièrement intéressant, en raison, d'une part de sa facilité de mise en oeuvre, et, d'autre part, du fait qu'il utilise des matières premières peu coûteuses, conduisant directement aux acides acryliques de formule (I) et ceci avec des rendements élevés.

Selon une caractéristique essentielle du procédé conforme à l'invention, celui-ci utilise comme matières premières les esters d'acides maloniques alkylés de formule (VIII) 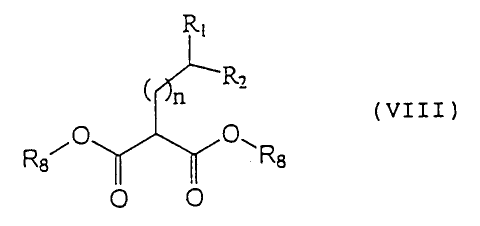 dans laquelle n, R₁ et R₂ ont la même signification que dans la formule (I) et R₈ représente une chaîne alkyle contenant de 1 à 4 atomes de carbone.

Les esters maloniques alkylés de formule (VIII) peuvent être obtenus en faisant réagir un halogénure de formule (IX) 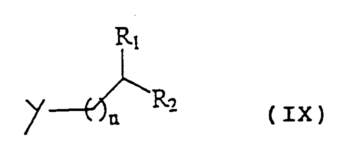 dans laquelle n, R₁ et R₂ ont la même signification que dans la formule (I) et Y représente un atome d'halogène, avec un ester d'acide malonique de formule (X) 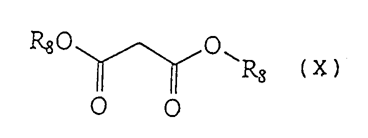 dans laquelle R₈ a la définition précitée, en présence d'une solution alcoolique d'un métal alcalin.

L'halogènure de formule (IX) utilisé pour effectuer la synthèse malonique des composés de formule (VIII) est, de préférence, un dérivé chloré, bromé ou iodé.

La solution alcoolique d'un métal alcalin en présence de laquelle s'effectue la synthèse malonique des dérivés de formule (VIII) peut être par exemple une solution de sodium dans l'éthanol ou une solution de sodium dans le méthanol.

Les esters maloniques de formule (VIII) peuvent être encore obtenus par une condensation de Knoevenagel d'un composé carbonylé de formule (XI) ou (XI') 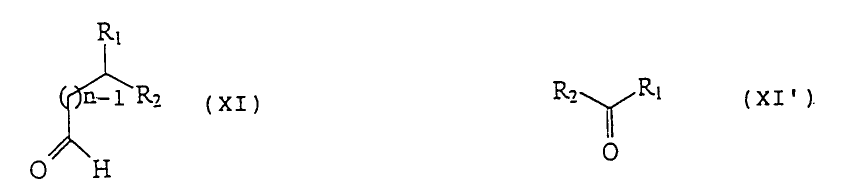 dans lesquelles R₁ et R₂ ont la même définition que dans la formule (I) et n varie de 1 à 10,
avec un ester d'acide malonique de formule (X) en présence d'une base et d'un acide carboxylique, en effectuant un entraînement azéotropique dans un solvant organique tel que le toluène, pour former les esters de formule (XII) ou (XII') 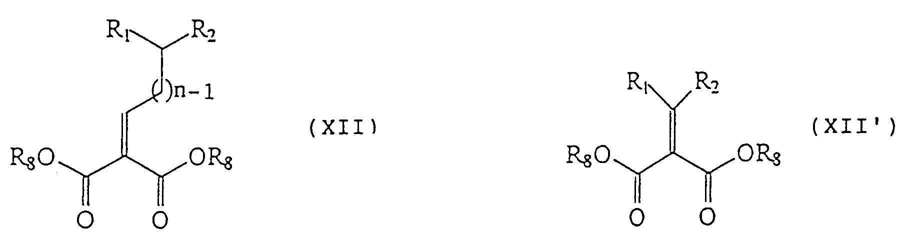 dans lesquelles R₁, R₂ et R₈ ont les significations précitées et n varie de 1 à 10.

La base utilisée pour la réaction de Knoevenagel est, de préférence, la pipéridine et l'acide carboxylique utilisé pour cette même réaction peut être par exemple l'acide acétique ou l'acide benzoïque.

On obtient les esters de formule (VIII) par hydrogénation catalytique des esters de formule (XII) ou (XII'). Le catalyseur d'hydrogénation peut être par exemple du charbon palladié.

Le procédé de synthèse des acides acryliques de formule (I) conforme à l'invention consiste donc, tout d'abord, à préparer les esters maloniques de formule (VIII), soit en partant d'un halogénure de formule (IX), soit en partant d'un composé carbonylé de formule (XI) ou (XI') par une réaction de Knoevenagel, comme il a été précédemment indiqué.

Les esters maloniques de formule (VIII) sont ensuite saponifiés par une solution aqueuse basique, telle qu'une solution aqueuse de soude pour conduire aux diacides de formule (XIII) 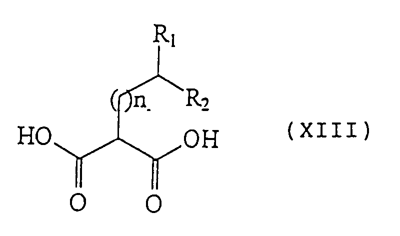 dans laquelle n, R₁ et R₂ ont la même définition que dans la formule (I).

Les diacides de formule (XIII) sont soumis à une réaction de Mannich avec une base organique et du formaldéhyde pour conduire aux acides acryliques de formule (I).

La base utilisée pour effectuer la réaction de Mannich est, de préférence, choisie parmi la diéthylamine, la diméthylamine ou la pipéridine.

Le procédé conforme à l'invention est plus particulièrement adapté à la préparation des acides acryliques de formule

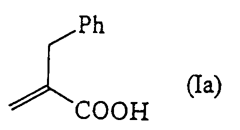

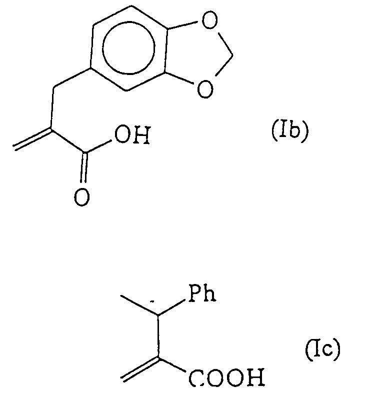

Appliqué à la préparation de l'acide acrylique (Ia) ou (Ib) et si l'on procède à la préparation de l'ester malonique (VIII) en partant d'un composé carbonylé (XI), le procédé conforme à l'invention consiste :
- à effectuer une condensation de Knoevenagel du benzaldéhyde (s'il s'agit de préparer l'acide (Ia)) ou du pipéronal (s'il s'agit de préparer l'acide (Ib)) avec un malonate de formule (X), en présence d'une base telle que la pipéridine et d'un acide organique tel que l'acide acétique dans un solvant tel que le toluène,
- à soumettre l'ester formé à une hydrogénation catalytique, en présence par exemple de charbon palladié,
- à saponifier le diester obtenu avec une solution aqueuse alcaline par exemple une solution aqueuse de soude,
- à libérer le diacide de formule (XIIIa) ou (XIIIb) 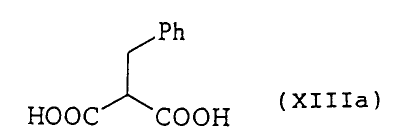 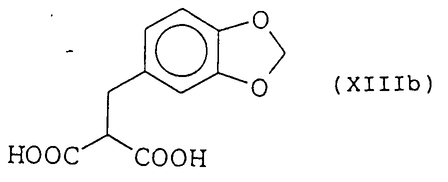 par acidification, puis à les extraire par un solvant tel que l'acétate d'éthyle,
- et à soumettre le diacide à l'action d'une base organique telle que la diéthylamine et du formaldéhyde pour former l'acide (Ia) ou (Ib).
Appliqué à la préparation de l'acide acrylique (Ic) et si l'on procède à la préparation de l'ester malonique (VIII) en partant d'un halogénure de formule (IX), le procédé conforme à l'invention consiste :
- à faire réagir un halogénure (IX) par exemple le 1-chloro-1-phényléthane avec un malonate de formule (X), en présence d'une solution alcoolique d'un métal alcalin, telle qu'une solution de sodium dans l'éthanol ou dans le méthanol,
- à saponifier le diester obtenu avec une solution aqueuse alcaline, par exemple une solution aqueuse de soude,
- à libérer par acidification le diacide de formule (XIIIc) 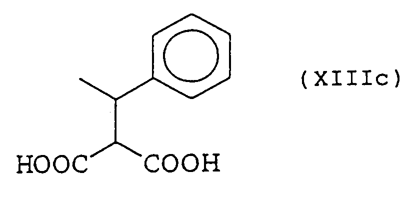 puis à l'extraire par un solvant tel que l'acétate d'éthyle,
- et à soumettre le diacide à l'action d'une base organique telle que la diéthylamine et du formaldéhyde pour former l'acide (Ic).

Les acides acryliques de formule (I) obtenus par le procédé conforme à la présente invention trouvent une utilisation particulièrement avantageuse dans la synthèse des N-(mercaptoacyl) amino-acides de formule (II). Ils sont particulièrement appropriés à la synthèse des dérivés d'amino-acides de formules (III), (IV), (V), (VI) et (VII).

La préparation des N-(mercaptoacyl) amino-acides à partir des acides acryliques est connue et est par exemple décrite dans le brevet européen n° 0 419 327.

Les N-(mercaptoacyl) amino-acides peuvent être obtenus par cette succession d'étapes :
- l'acide acrylique est soumis à une addition de Michael avec un dérivé soufré de formule R₅-SH pour former l'acide de formule (XIV) 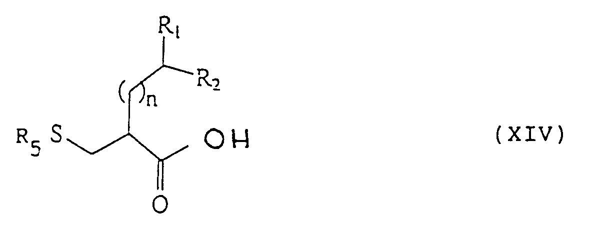 où R₁, R₂ et R₅ ont les significations qui ont été données dans la formule (II)
- l'acide de formule (XIV) est éventuellement dédoublé,
- l'acide de formule (XIV) sous forme racémique ou optiquement pure est couplé avec un amino-ester de formule (XV) 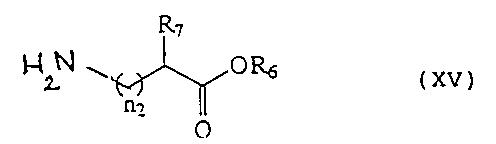 où R₆ et R₇ et n₂ ont les significations qui ont été données dans la formule (II), en présence d'un agent de couplage tel que le dicyclohexylcarbodiimide, pour former les dérivés de 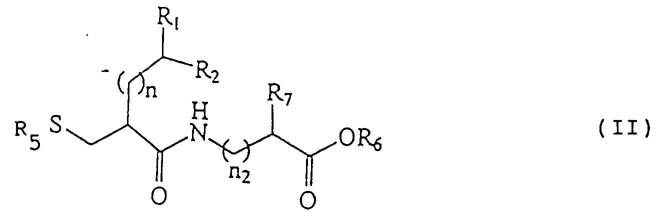 formule (II)

Il va être donné, ci-dessous, à titre non limitatif, quelques exemples illustrant la mise en oeuvre du procédé conforme à l'invention.

### Exemple 1 : Acide benzylacrylique (Ia)

### Voie A : Synthèse à partir du malonate de diéthyle

### Stade 1 : benzylidène malonate de diéthyle.

Dans un erlenmeyer tricol d'un litre, on introduit 60 g (565,39 mmol) de benzaldéhyde, 90,56 g (565,39 mmol) de malonate de diéthyle, 3,85 g (45,21 mmol) de pipéridine, 130 ml de toluène et 2,71 g (45,16 mmol) d'acide acétique.

On équipe le tricol d'un Dean-Stark et d'un réfrigérant. On agite et on porte au reflux pendant 3h 30. On récupère 12,7 ml d'eau, puis on laisse le milieu réactionnel revenir à température ambiante.

### Stade 2 : benzylmalonate de diéthyle

La solution précédente est transvasée dans un hydrogénateur de 450 ml. On introduit 3 g de charbon palladié à 10% et on purge le montage 3 fois avec de l'hydrogène. On introduit de l'hydrogène jusqu'à une pression de 15 bars. La température initiale est de 27°C.

Après 1h 15 d'agitation, la température est de 39°C et la pression est à 8 bars.

On augmente à nouveau la pression jusqu'à 12 bars avec de l'hydrogène et on chauffe à environ 55°C pendant 2 heures.

On refroidit ensuite jusqu'à température ambiante, puis on décomprime.

La solution est filtrée sur un verre fritté de porosité n° 4.

### Stade 3 : acide benzylmalonique (XIIIa)

On transvase la solution précédente dans un erlenmeyer tricol d'un litre et on ajoute successivement 117 ml (1,412 moles) d'une solution aqueuse de soude à 35% et 117 ml d'eau.

On agite énergiquement et on porte au reflux pendant 2h 45.

On revient ensuite à température ambiante. Le mélange réactionnel est transféré dans une ampoule à décanter et la phase organique est éliminée.

La phase aqueuse basique est refroidie à environ 10°C, agitée énergiquement et acidifiée avec 115 ml d'HCl à 35% (pH = 1).

On l'extrait à l'acétate d'éthyle : 1 fois avec 100 ml et 1 fois avec 70 ml. Ces deux phases sont réunies.

### Stade 4 : acide benzylacrylique (Ia)

La solution précédente est refroidie à environ 10°C.

On ajoute, sous agitation, successivement 58,3 ml (564,63 mmol) de diéthylamine sans dépasser 30°C dans le milieu et 26,8 g (848,66 mmol) de paraformaldéhyde. On porte au reflux pendant 30 minutes. Le dégagement de CO₂ est alors terminé et la solution est limpide.

La température est de 61°C au début du reflux et atteint 72°C à la fin. On refroidit ensuite à environ 10°C, on dilue avec 50 ml d'eau et on acidifie avec 50 ml d'HCl 35% (pH = 1) sans dépasser 20°C dans le milieu.

On transfère le mélange dans une ampoule à décanter et on élimine la phase aqueuse.

La phase acétate d'éthyle est concentrée à l'évaporateur rotatif jusqu'à l'obtention d'une huile. On ajoute alors 100 ml d'eau et on termine l'évaporation de l'acétate d'éthyle. On observe une précipitation de l'acide. La suspension est agitée, refroidie à 10°C, filtrée et lavée deux fois avec 100 ml d'eau.

Le sel est séché sur P₂O₅ et KOH jusqu'à masse constante.

On obtient 77,3 g de sel blanc.
Rendement global pour ces quatres stades : 84%.
Point de fusion : 69°C (Kofler).
RMN¹H (CDCl₃) : (200 MHz) : 11,7 (singulet large, 1H) ; 7,4 à 7,15 (massif, 5H) ; 6,45 (singulet, 1H) ; 5,6 (doublet, 1H, J = 1,2 Hz) ; 3,65 (singulet, 2H).
Voie B : Synthèse à partir du malonate de diméthyle

### Stade 1 : benzylidène malonate de diméthyle

Dans un erlenmeyer tricol d'un litre, on introduit successivement 60 g (565,39 mmol) de benzaldéhyde, 77 g de malonate de diméthyle (565,32 mmol), 3,85 g de pipéridine (45,21 mmol), 130 ml de toluène et 2,71 g (45,16 mmol) d'acide acétique.

On équipe le tricol d'un Dean-Stark et on porte à reflux, sous agitation, pendant 3 heures.

On reccueille 15 ml d'eau, puis on laisse le milieu réactionnel revenir à température ambiante.

### Stade 2 : benzylmalonate de diméthyle

La solution précédente est transférée dans un hydrogénateur de 450 ml. On introduit 3 g de charbon palladié à 10% et on purge le montage 3 fois avec de l'hydrogène. On agite et on introduit de l'hydrogène jusqu'à une pression de 15 bars.

La température initiale est de 22°C.

Après 20 minutes d'agitation, la température atteint 56°C et la pression est à 6 bars. On augmente à nouveau la pression jusqu'à une pression de 15 bars avec de l'hydrogène et on maintient une température d'environ 55°C pendant 1h 30. On refroidit ensuite jusqu'à température ambiante, puis on décomprime. La solution est filtrée sur un verre fritté de porosité n° 4.

### Stade 3 : acide benzylmalonique (XIIIa)

On transvase la solution précédente dans un erlenmeyer tricol d'un litre et on ajoute successivement 117 ml (1,412 moles) de NaOH à 35% et 117 ml d'eau.

On agite de façon énergique et on porte au reflux pendant 3 heures.

On revient à température ambiante et on verse le mélange dans une ampoule à décanter. La phase organique est éliminée et la phase aqueuse refroidie, agitée vivement et acidifiée avec 115 ml d'HCl 35% (pH = 1).

On l'extrait à l'acétate d'éthyle : 1 fois avec 100 ml et 1 fois avec 70 ml. Ces deux phases sont réunies.

### Stade 4 : acide benzylacrylique (Ia)

La solution précédente est refroidie par un bain de glace-eau. On agite et on ajoute successivement 58,3 ml (564,63 mmol) de diéthylamine sans dépasser 30°C dans le milieu et 26,8 g (848,66 mmol) de paraformaldéhyde.

On porte au reflux pendant 30 minutes. Le dégagement de CO₂ est alors terminé et le milieu est devenu limpide. On refroidit ensuite avec un bain de glace-eau, on dilue avec 50 ml d'eau et on acidifie avec 50 ml d'HCl 35% (pH = 1). On élimine la phase aqueuse acide et on concentre la phase organique à l'évaporateur rotatif jusqu'à l'obtention d'une huile. On ajoute alors 100 ml d'eau et on termine l'évaporation de l'acétate d'éthyle. On observe une précipitation de l'acide.

La suspension est agitée, refroidie par un bain de glace-eau jusqu'à environ 15°C et filtrée. On lave le précipité deux fois avec 100 ml d'eau. Le précipité est séché sur P₂O₅ et KOH jusqu'à masse constante. On obtient 71,86 g de solide blanc.
Rendement global pour ces quatre stades : 78%
Point de fusion : 69°C (Kofler)

### Exemple 2 : acide pipéronylacrylique (Ib)

### Stade 1 : pipéronylidène malonate de diéthyle

Dans un erlenmeyer tricol d'un litre, on introduit 50 g (333,04 mmol) de pipéronal, 53,34 g (333,04 mmol) de malonate de diéthyle, 2,26 g (26,59 mmol) de pipéridine, 110 ml de toluène et 1,6 g (26,66 mmol) d'acide acétique.

On équipe le tricol d'un Dean-Stark et d'un réfrigérant. On agite et on porte au reflux pendant 3h 30. On récupère 7 ml d'eau, puis on laisse le milieu réactionnel revenir à température ambiante.

### Stade 2 : pipéronylmalonate de diéthyle

La solution précédente est transvasée dans un hydrogénateur de 450 ml.

On introduit 2,5 g de charbon palladié à 10%, et on purge le montage 3 fois avec de l'hydrogène. On agite et on introduit de l'hydrogène jusqu'à une pression de 15 bars. La température initiale est de 20°C.

Après 1 h d'agitation, la température est de 29°C et la pression est à 10 bars. On augmente à nouveau la pression jusqu'à 15 bars avec de l'hydrogène et on chauffe à environ 55°C pendant 2h 30.

On refroidit ensuite jusqu'à température ambiante, puis on décomprime.

On filtre sur un verre fritté de porosité n° 4 et on rince avec 20 ml de toluène.

### Stade 3 : acide pipéronylmalonique (XIIIb)

La solution précédente est transvasée dans un erlenmeyer tricol d'un litre et on ajoute successivement 69 ml de NaOH 35% (833,17 mmol) et 69 ml d'eau.

On agite énergiquement et on porte au reflux pendant 3 heures.

On revient ensuite à température ambiante et on verse le mélange dans une ampoule à décanter. On élimine la phase organique, on refroidit la phase aqueuse par un bain glace-eau et on l'agite énergiquement. On ajoute successivement 90 ml d'acétate d'éthyle et 70 ml d'HCl à 35% (pH = 1).

On transfère dans une ampoule à décanter et on récupère la phase organique.

La phase aqueuse est de nouveau extraite avec 60 ml d'acétate d'éthyle. Ces deux phases sont réunies.

### Stade 4 : acide pipéronylacrylique (Ib)

La solution précédente est introduite dans un erlenmeyer de 500 ml et agitée vivement.

On ajoute successivement 34,3 ml (332,19 mmol) de diéthylamine (addition en 2 minutes), la température atteignant alors 50°C, et 15,7 g (497,16 mmol) de paraformaldéhyde.

On observe alors une précipitation du milieu. On porte au reflux pendant 30 minutes. Le dégagement de CO₂ est alors terminé et le milieu est devenu limpide.

La température est de 61°C au début du reflux et atteint 72°C à la fin. On refroidit avec un bain de glace, on dilue avec 40 ml d'eau et on acidifie avec 30 ml d'HCl 35%. On observe alors une précipitation. Le précipité est filtré et le filtrat est transféré dans une ampoule à décanter. Après avoir éliminé la phase aqueuse, on dilue la phase acétate d'éthyle avec 60 ml d'eau et on concentre sous vide à l'évaporateur rotatif. On observe alors une précipitation. La suspension est refroidie à environ 10°C et filtrée. On réunit les deux précipités qui sont lavés deux fois avec 100 ml d'eau. On obtient 77,06 g de solide que l'on sèche sous vide sur P₂O₅ et KOH jusqu'à masse constante.

On obtient 58,51 g de solide blanc.
Rendement globale pour ces quatre stades : 85 %.
Point de fusion : 130°C (Kofler).
RMN¹H (CDCl₃) : 200 MHz : 10 (singulet large, 1H) ; 6,8 à 6,6 (massif, 3H) ; 6,85 (doublet, 1H, J = 0,4 Hz) ; 5,9 (singulet, 2H) ; 5,6 (doublet, 1H, J = 1,5 Hz) ; 3,5 (singulet, 2H).

### Exemple 3 : Acide 2-(1-phényléthyl) acylique (Ic)

### Stade 1 : (1-phényléthyl)malonate de diéthyle.

On introduit dans un erlenmeyer tricol 58,7 g (417,79 mmol) de 1-chloro-1-phényléthane et 280 g (1,75 moles) de malonate de diéthyle. On ajoute sous agitation une solution préparée à partir de 25 g (1,086 moles) de sodium et de 640 ml d'éthanol, puis on porte ce mélange au reflux pendant 5 heures. On évapore l'éthanol à l'évaporeur rotatif, puis on reprend le résidu à l'eau (150 ml) et à l'éther éthylique (300 ml). On élimine la phase aqueuse et on lave la phase organique à l'eau jusqu'à pH neutre.

La phase éthérée est séchée sur sulfate de magnésium, filtrée et concentrée.

On obtient un résidu huileux que l'on distille à la pompe à palettes, afin d'éliminer l'excès de malonate de diéthyle (60-70°C sous 0,2 mm Hg).

Le résidu de distillation contient le (1-phényléthyl) malonate de diéthyle.
Masse = 102,5 g.
Rendement = 92%.

### Stade 2 : acide (1-phényléthyl) malonique (XIIIc)

On ajoute à 92,44 g (350,15 mmol) de (1-phényléthyl) malonate de diéthyle (stade 1), 72,5 ml (875,43 mmol) d'une solution de soude à 35% et 290 ml d'eau.

On porte au reflux pendant 3 heures. On laisse le milieu réactionnel revenir à température ambiante et on distille l'éthanol à l'évaporateur rotatif.

La phase aqueuse est refroidie et acidifiée avec de l'HCl concentré jusqu'à pH = 1.

On l'extrait avec de l'éther éthylique (2 fois 200 ml). Les phases éthérées sont réunies, séchées sur sulfate de magnésium, filtrées et concentrées. On obtient ainsi l'acide (1-phényléthylmalonique) sous forme d'un solide blanc.
Masse = 70,7 g
Rendement = 97 %
Fusion = 136°C (Köfler).

### Stade 3 : acide 2-(1 phényléthyl) acrylique (Ic)

A une solution de 70,7 g (339,90 mmol) d'acide (1-phényléthyl)malonique provenant du stade 2 dans 450 ml d'acétate d'éthyle, on ajoute successivement 35,1 ml (339,94 mmol) de diéthylamine et 16,1 g (509,83 mmol) de paraformaldéhyde.

On porte au reflux pendant 30 minutes. Le dégagement de gaz carbonique est alors terminé et le milieu est devenu limpide.

On refroidit avec un bain de glace, on dilue avec 40 ml d'eau et on acidifie jusqu'à pH = 1 avec de l'HCl à 35%. La phase aqueuse est décantée et la phase organique diluée avec 60 ml d'eau et concentrée sous vide. Le précipité est filtré et lavé deux fois avec 100 ml d'eau.

On obtient 59,17 g de solide que l'on sèche sous vide sur P₂0₅ jusqu'à masse constante.
Masse = 53,77 g
Rendement = 89%
Fusion = 113°C
RMN¹H (CDCl₃) = 11,2 (singulet large, 1H) ; 7,45 à 7,15 (massif, 5H) ; 6,5 (singulet 1H) ; 5,75 (singulet, 1H) ; 4,05 (quadruplet, 1H, J = 7Hz) ; 1,5 (doublet, 3H, J = 7Hz).

### Exemples 4 à 9

En suivant le mode opératoire de l'exemple 3, mais en utilisant pour les exemples 6, 7 et 8 les malonates de diéthyle commerciaux, on obtient les acides acryliques (I) :

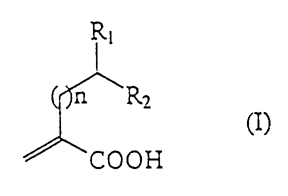

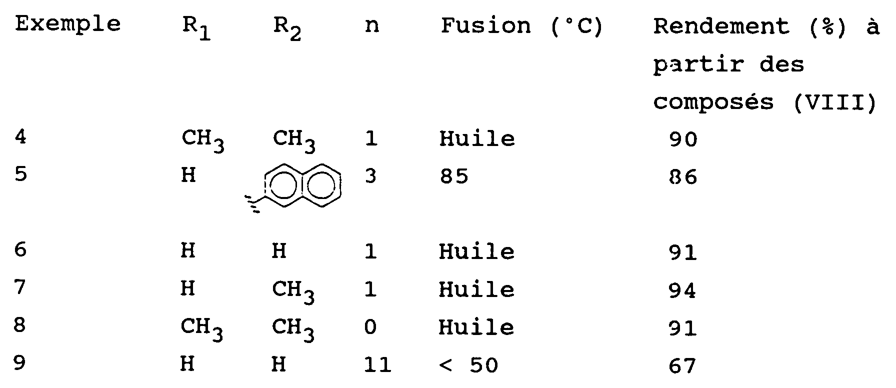

### Exemple 10 : N-(RS)-[2-acétylthiométhyl-1-oxo-3-phénylpropyl]- glycinate de benzyle (III)

### Stade 1 : acide 2-acétylthiométhyl-3-phényl propanoïque

Dans un ballon muni d'un réfrigérant et d'une garde à CaCl₂, on place 10 g d'acide benzylacrylique Ia (61,7 mmol) et 7,1 ml d'acide thioacétique (1,6 eq).

Le mélange est chauffé pendant 24 H à 70°C sous agitation.

On évapore sous vide (1 mm, 60°C) l'excès d'acide thioacétique.

Le résidu pâteux jaune est repris à trois reprises par 50 ml d'éther. A chaque fois l'éther est évaporé à pression ordinaire, puis le résidu séché sous vide.

Ces étapes ont pour but l'élimination des traces restantes d'acide thioacétique.

On obtient 14 g d'une huile jaune très épaisse (Rdt = 95%).

### Stade 2 : N-(RS)-[2-acétylthiométhyl-1-oxo-3-phénylpropyl]-glycinate de benzyle (III)

Dans un ballon muni d'une garde à CaCl₂ et d'un agitateur magnétique, on place 10 g d'acide acétylthio-3 benzyl-2 propanoïque (42 mmol) en solution dans 70 ml de THF anhydre.

On refroidit le ballon à environ 0-5°C par un bain de glace et on ajoute successivement le sel de paratoluènesulfonate du glycinate de benzyle (42 mmol) et 5,75 ml de triéthylamine (42 mmol) dans 80 ml de chloroforme, une solution de 6,3 g (42 mmol) d'hydroxybenzotriazole dans 60 ml de THF anhydre et une solution de 8,65 g (42 mmol) de dicyclohexylcarbodiimide dans 50 ml de CHCl₃.

On agite le mélange 1 H à 0°C, puis environ 6 H à température ambiante.

Le précipité de DCU est filtré et on évapore à sec. Le résidu pâteux est repris par l'acétate d'éthyle (100 ml). Le DCU qui a à nouveau précipité, est filtré. La phase organique est successivement lavée par 1 fois 20 ml d'eau, 3 fois 20 ml d'une solution saturée de NaHCO₃, 1 fois 20 ml d'eau, 1 fois 20 ml d'une solution saturée de NaCl.

On sèche sur sulfate de sodium et on évapore à sec.

On obtient un résidu solide blanc, recristallisable dans l'éther.

Exemple pour l'ester benzylique :
- Poids: = 14,6 g
- Rdt: = 90 %
- F: = 89°C

CCM (gel de silice) Rf (CHCl₃/MeoH/eau : 9/1/sat) = 0,80

## Revendications

1. Procédé de synthèse d'acides acryliques α-substitués de formule (I) 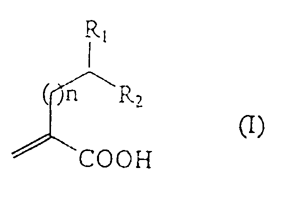 dans laquelle
R₁ représente un atome d'hydrogène ; un groupe alkyle ; un groupe cycloalkyle ; un groupe phényle éventuellement mono- ou polysubstitué par un atome d'halogène, un groupe trifluorométhyle, un groupe nitro, un groupe cyano, un groupe amino, un groupe diméthylamino, un groupe hydroxy, un groupe alcoxy en C₁-C₆, un groupe phénoxy, un groupe benzyloxy, un groupe méthylthio, un groupe phényle, un groupe alkyle en C₁-C₆, un groupe phényl(alkylène en C₁-C₆) ; un groupe alpha et bêta naphtyle ; un groupement 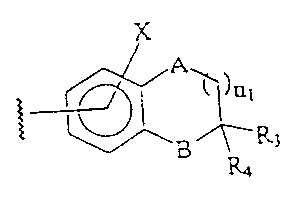 où
A est un groupe méthylène, un atome d'oxygène, un atome de soufre ou un atome d'azote,
B représente un des groupe ou atomes précités pour la définition de A, n₁ est égal à 0 ou 1,
X représente un atome d'hydrogène, un atome d'halogène, un groupe hydroxy, un groupe alcoxy en C₁-C₆ ou un groupe trifluorométhyle,
R₃ représente un atome d'hydrogène, un groupe phényle, un groupe alkyle en C₁-C₆, un atome d'halogène ou un groupe trifluorométhyle,
R₄ représente également un atome d'hydrogène ou l'un des groupes précités pour la définition de R₃,
R₂ représente un atome d'hydrogène ou l'un des groupes précités pour la définition de R₁ et
n varie de 0 à 10, caractérisé en ce que, successivement,
a) on prépare les esters d'acides maloniques alkylés de formule (VIII) 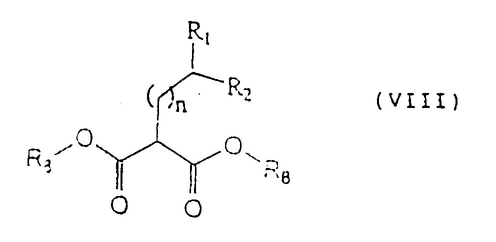 dans laquelle n, R₁ et R₂ ont la même signification que dans la formule (I) et R₈ représente une chaîne alkyle contenant de 1 à 4 atomes de carbone,
les esters d'acides maloniques alkylés étant obtenus
. soit en faisant réagir un halogénure de formule (IX) 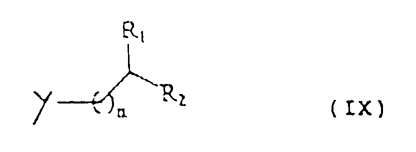 dans laquelle n, R₁ et R₂ ont la même signification que dans la formule (I) et Y représente un atome d'halogène, sur un ester d 'acide malonique de formule (X) 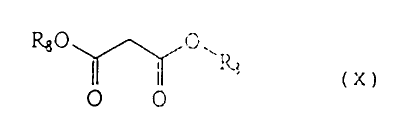 dans laquelle R₈ a la définition précitée, en présence d'une solution alcoolique d'un métal alcalin,
. soit par une condensation de Knoevenagel d'un composé carbonylé de formule ( XI) ou (XI') 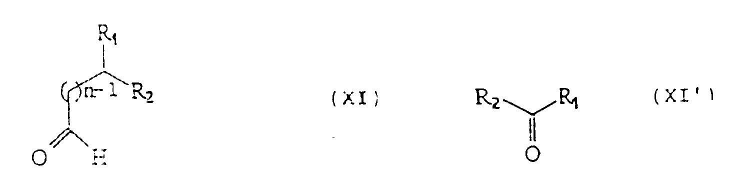 dans lesquelles R₁ et R₂ ont la même définition que dans la formule (I) et n varie de 1 à 10, avec un ester d'acide malonique de formule (X) en présence d'une base et d'un acide carboxylique, pour conduire aux esters de formule (XII) ou (XII') 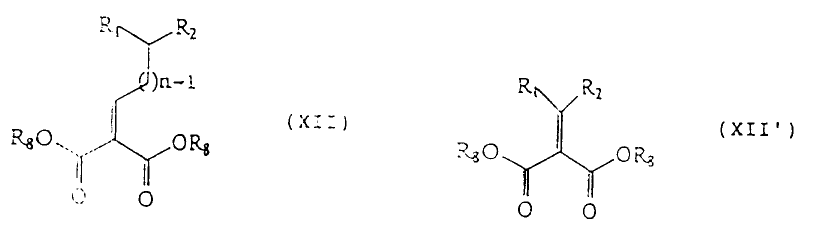 dans lesquelles R₁, R₂ et R₈ ont les significations précitées et n varie de 1 à 10, les esters de formule (XII) ou (XII') étant ensuite soumis à une hydrogénation catalytique pour former les esters de formule (VIII),
b) on saponifie les esters de formule (VIII), en présence d'une solution aqueuse basique pour former les diacides de formule (XIII) 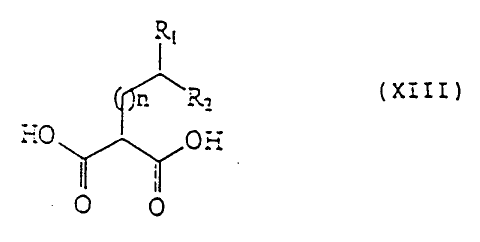 dans laquelle n, R₁ et R₂ ont la même définition que dans la formule (I),
c) on soumet les diacides de formule (XIII) à une réaction de Mannich avec une base organique et du formaldéhyde pour former les acides acryliques de formule (I).

2. Procédé selon la revendication 1, caractérisé en ce que l'halogénure de formule (IX) est un dérivé chloré, bromé ou iodé.

3. Procédé selon l'une des revendications 1 et 2, caractérisé en ce que la solution alcoolique d'un métal alcalin est une solution de sodium dans l'éthanol ou une solution de sodium dans le méthanol.

4. Procédé selon l'une quelconque des revendications 1 à 3, caractérisé en ce que la base utilisée pour la réaction de Knoevenagel est la pipéridine.

5. Procédé selon l'une quelconque des revendications 1 à 4, caractérisé en ce que l'acide carboxylique utilisé pour la réaction de Knoevenagel est choisi parmi l'acide acétique et l'acide benzoïque.

6. Procédé selon l'une quelconque des revendications 1 à 5, caractérisé en ce que l'on effectue l'hydrogénation catalytique des composés de formules (XII) et (XII') en utilisant comme catalyseur du charbon palladié.

7. Procédé selon l'une quelconque des revendications 1 à 6, caractérisé en ce que la solution aqueuse basique utilisée pour la saponification des diesters de formule (VIII) est une solution aqueuse de soude.

8. Procédé selon l'une quelconque des revendications 1 à 7, caractérisé en ce que la base utilisée pour la réaction de Mannich est choisie parmi la diéthylamine, la diméthylamine et la pipéridine.

9. Procédé selon l'une quelconque des revendications 1 à 8, caractérisé en ce qu'il consiste, lorsqu'il est appliqué à la préparation de l'acide acrylique de formule 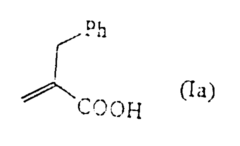 à effectuer une condensation de Knoevenagel d'un composé carbonylé de formule (XI), le benzaldéhyde, avec un malonate de formule (X), en présence d'une base telle que la pipéridine et d'un acide carboxylique tel que l'acide acétique, dans un solvant tel que le toluène,
- à soumettre à une hydrogénation catalytique les esters obtenus,
- à saponifier les diesters par une solution aqueuse alcaline, telle qu'une solution aqueuse de soude,
- à libérer par acidification le diacide de formule (XIIIa) 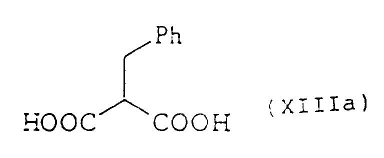 et à l'extraire par un solvant organique tel que l'acétate d'éthyle,
- et à soumettre la solution à l'action d'une base telle que la diéthylamine et du formaldéhyde pour donner l'acide de formule (Ia).

10. Procédé selon l'une quelconque des revendications 1 à 8, caractérise en ce qu'il consiste, lorsqu'il est appliqué à la préparation de l'acide acrylique de formule 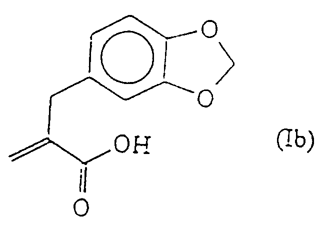
- à condenser un composé carbonylé de formule (XI), le pipéronal, selon une réaction de Knoevenagel avec un malonate de formule (X), en présence d'une base telle que la pipéridine et d'un acide organique tel que l'acide acétique dans un solvant organique tel que le toluène,
- à soumettre à une hydrogénation catalytique les esters formés,
- à saponifier les diesters par une solution aqueuse alcaline telle qu'une solution de soude,
- à libérer par acidification le diacide de formule (XIIIb) 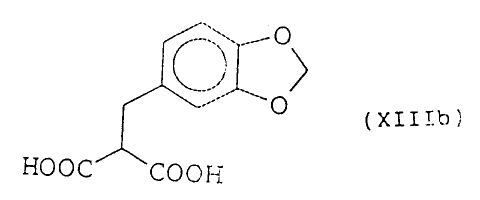 puis à l'extraire par un solvant organique tel que l'acétate d'éthyle,
- et à soumettre la solution à l'action d'une base telle que la diéthylamine et du formaldéhyde pour donner l'acide de formule (Ib).

11. Procédé selon l'une quelconque des revendications 1 à 8, caractérisé en ce qu'il consiste, lorsqu'il est appliqué à la préparation de l'acide acrylique de formule 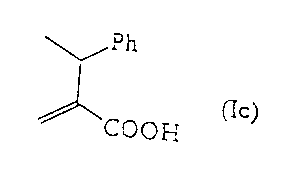
- à faire réagir un halogènure de formule (IX) tel qu'un dérivé chloré dans lequel R₁ est un groupement phényle, R₂ est un groupement méthyle, Y désigne le chlore et n = 0, avec un ester malonique de formule (X) en présence d'une solution alcoolique d'un métal alcalin telle qu'une solution de sodium dans l'éthanol ou dans le méthanol,
- à saponifier le diester obtenu par une solution aqueuse alcaline, telle qu'une solution aqueuse de soude,
- à libérer par acidification le diacide de formule (XIIIc) 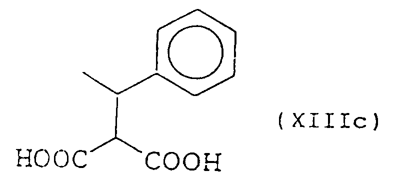 et à l'extraire par un solvant organique tel que l'acétate d'éthyle,
- et à soumettre la solution à l'action d'une base telle que la diéthylamine et du formaldéhyde pour donner l'acide de formule (Ic).

12. Procédé pour la synthèse de N-(mercaptoacyl) amino-acides de formule (II) 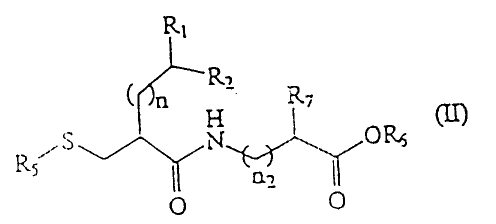 dans laquelle
R₁ et R₂ ont la même signification que dans la formule (I)selon la revendication 1,
n varie de 0 à 10,
R₅ représente un atome d'hydrogène, un radical acyle aliphatique linéaire ou ramifié ou un radical acyle aromatique,
R₆ représente un atome d'hydrogène, un radical alkyle en C₁-C₆, un radical phényle ou un groupe phényl(alkylène en C₁-C₆),
n₂ varie de 0 à 10,
R₇ représente un atome d'hydrogène ; un groupe alkyle en C₁-C₆ ; un groupe hydroxyalkylène en C₁-C₆ ; un groupe phényle ; un groupe phényl(alkylène en C₁-C₆) ; un groupe hydroxyphényl(alkylène en C₁-C₆) ; un groupe aminoalkylène en C₁-C₆ ; un groupe guanidino(alkylène en C₁-C₆) ; un groupe mercaptoalkylène en C₁-C₆ ; un groupe (alkyle en C₁-C₆) thioalkylène en C₁-C₆ ; un groupe imidazolyl(alkylène en C₁-C₆) ; un groupe indolyl(alkylène en C₁-C₆) ; un groupe carbamyl(alkylène en C₁-C₆), un groupe carboxy(alkylène en C₁-C₆), procédé dans lequel:
- un acide de formule (I) est préparé selon l'une quelconque des revendications 1-11,
- l'acide de formule (I) ainsi obtenu est soumis à une addition de Michael avec un dérivé soufré de formule R₅-SH pour former l'acide de formule (XIV) 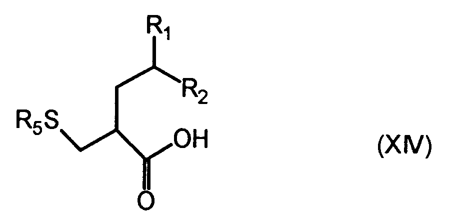
où R₁, R₂ et R₅ ont les significations qui ont été données dans la formule (II)
- l'acide de formule (XIV) est éventuellement dédoublé,
- l'acide de formule (XIV) sous forme racémique ou optiquement pure est couplé avec un amino-ester de formule (XV) 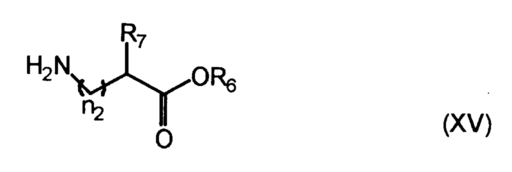
où R₆, R₇ et n₂ ont les significations qui ont été données dans la formule (II), pour former les dérivés de formule (II).

13. Procédé selon la revendication 12, caractérisé en ce que l'on prépare l'acide acrylique de formule (Ia) selon la revendication 9 pour la synthèse des dérivés de formule (II).

14. Procédé selon la revendication 13, caractérisé en ce que l'on prépare l'acide acrylique de formule (Ia) selon la revendication 9 pour la synthèse du N-(RS)-[2-acétylthiométhyl-1-oxo-3-phénylpropyl]-glycinate de benzyle.

15. Procédé selon la revendication 13, caractérisé en ce que l'on prépare l'acide acrylique de formule (Ia) selon la revendication 9 pour la synthèse du N-(S)-[2-acétylthiométhyl-1-oxo-3-phénylpropyl]-glycinate de benzyle.

16. Procédé selon la revendication 13, caractérisé en ce que l'on prépare l'acide acrylique de formule (Ia) selon la revendication 9 pour la synthèse du N-(R)-[2-acétylthiométhyl-1-oxo-3-phénylpropyl]-glycinate de benzyle.

17. Procédé selon la revendication 12, caractérisé en ce que l'on prépare l'acide acrylique de formule (Ib) selon la revendication 10 pour la synthèse des dérivés de formule (II).

18. Procédé selon la revendication 17, caractérisé en ce que l'on prépare l'acide acrylique de formule (Ib) selon la revendication 10 pour la synthèse du N-(S)-[2-acétylthiométhyl-1-oxo-3 (3,4-méthylènedioxyphényl)propyl]-(S)-alaninate de benzyle.

19. Procédé selon la revendication 12, caractérisé en ce que l'on prépare l'acide acrylique (Ic) selon la revendication 11 pour la synthèse des dérivés de formule (II).

20. Procédé selon la revendication 19, caractérisé en ce que l'on prépare l'acide acrylique (Ic) selon la revendication 11 pour la synthèse du N-[(2S, 3R)-2-benzoylthiométhyl-1-oxo-3-phénylbutyl]-(S)-alanine.

## Claims

1. Process for the synthesis of α-substituted acrylic acids of formula (I) 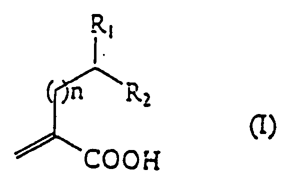 in which
R₁ represents a hydrogen atom; an alkyl group; a cycloalkyl group; a phenyl group possibly mono- or polysubstituted by a halogen atom, a trifluoromethyl group, a nitro group, a cyano group, an amino group, a dimethylamino group, a hydroxy group, a C₁-C₆ alkoxy group, a phenoxy group, a benzyloxy group, a methylthio group, a phenyl group, a C₁-C₆ alkyl group, a phenyl(C₁-C₆ alkylene) group; an alpha and beta naphthyl group; a group 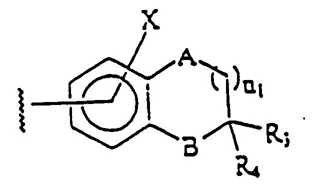 where
A is a methylene group, an oxygen atom, a sulphur atom or a nitrogen atom,
B represents one of the groups or atoms mentioned above for the definition of A,
n₁ is equal to 0 or 1,
X represents a hydrogen atom, a halogen atom, a hydroxy group, a C₁-C₆ alkoxy group or a trifluoromethyl group,
R₃ represents a hydrogen atom, a phenyl group, a C₁-C₆ alkyl group, a halogen atom or a trifluoromethyl group,
R₄ likewise represents a hydrogen atom or one of the groups mentioned above for the definition of R₃,
R₂ represents a hydrogen atom or one of the groups mentioned above for the definition of R₁, and
n varies from 0 to 10,
characterised in that successively
a) the alkylated malonic acid esters of formula (VIII) are prepared 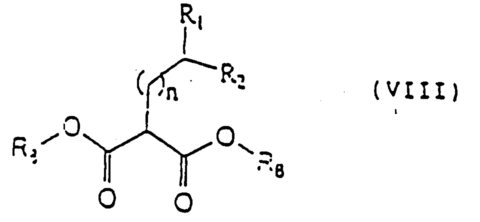 in which n, R₁ and R₂ have the same meaning as in formula (I) and R₈ represents an alkyl chain containing from 1 to 4 carbon atoms,
the alkylated malonic acid esters being obtained
either by causing a halide of formula (IX) 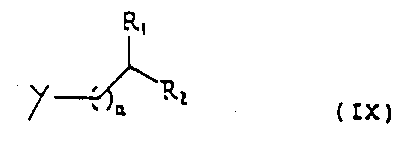 in which n, R₁ and R₂ have the same meaning as in formula (I) and Y represents a halogen atom, is made to react on a malonic acid ester of formula (X) 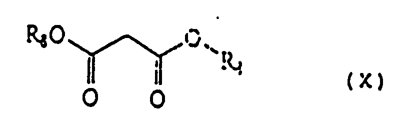 in which R₈ has the definition mentioned above, in the presence of an alcoholic solution of an alkaline metal,
or by Knoevenagel condensation of a carbonyl compound of formula (XI) or (XI') 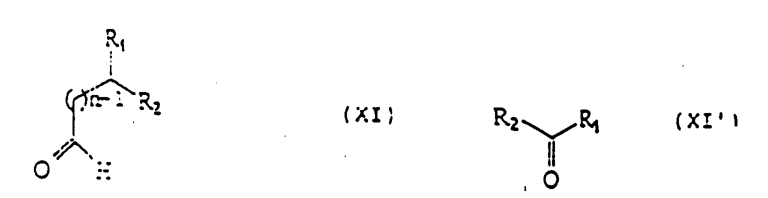 in which R₁ and R₂ have the same definition as in formula (I) and n varies from 1 to 10, with a malonic acid of formula (X) in the presence of a base and a carboxylic acid, in order to lead to the esters of formula (XII) or (XII') 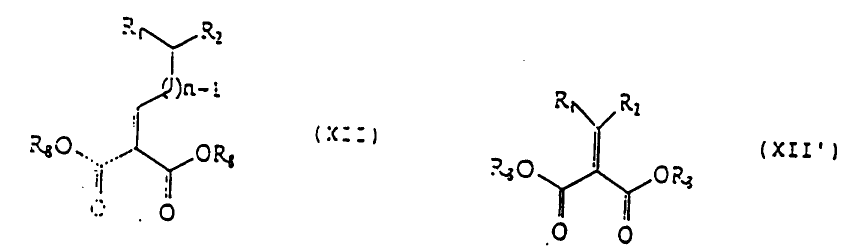 in which R_{1,} R₂ and R₈ have the meanings mentioned above and n varies from 1 to 10, the esters of formula (XII) or (XII') then being subjected to catalytic hydrogenation in order to form the esters of formula (VIII), b) the esters of formula (VIII) are saponified in the presence of a basic aqueous solution in order to form the diacids of formula (XIII) 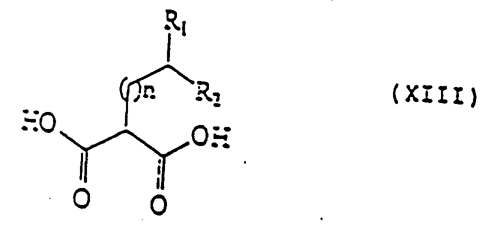 in which n, R₁ and R₂ have the same definition as in formula (I),
c) the diacids of formula (XIII) are subjected to a Mannich reaction with an organic base and formaldehyde in order to form the acrylic acids of formula (I).

2. Process as claimed in Claim 1, characterised in that the halide of formula (IX) is a chlorine, bromine or iodine derivative.

3. Process as claimed in one of Claims 1 and 2, characterised in that the alcoholic solution of an alkaline metal is a solution of sodium in ethanol or a solution of sodium in methanol.

4. Process as claimed in any one of Claims 1 to 3, characterised in that the base used for the Knoevenagel reaction is piperidine.

5. Process as claimed in any one of Claims 1 to 4, characterised in that the carboxylic acid used for the Knoevenagel reaction is chosen from amongst acetic acid and benzoic acid.

6. Process as claimed in any one of Claims 1 to 5, characterised in that the catalytic hydrogenation of the compounds of formulae (XII) and (XII') is carried out using palladinised carbon as catalyst.

7. Process as claimed in any one of Claims 1 to 6, characterised in that the basic aqueous solution used for the saponification of the diesters of formula (VIII) is an aqueous solution of soda.

8. Process as claimed in any one of Claims 1 to 7, characterised in that the base used for the Mannich reaction is chosen from amongst diethylamine, dimethylamine and piperidine.

9. Process as claimed in any one of Claims 1 to 8, characterised in that when applied to the preparation of the acrylic acid of formula 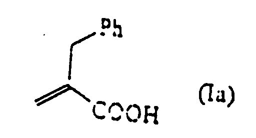 it consists of
- effecting a Knoevenagel condensation of a carbonyl compound of formula (XI), benzaldehyde, with a malonate of formula (X) in the presence of a base such as piperidine and a carboxylic acid such as acetic acid in a solvent such as toluene,
- subjecting the esters obtained thereby to a catalytic hydrogenation,
- saponification of the diesters by an alkaline aqueous solution such as an aqueous solution of soda,
- liberation by acidification of the diacid of formula (XIIIa) 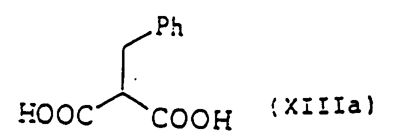 and extracting it with an organic solvent such as ethyl acetate,
- and subjecting the solution to the action of a base such as diethylamine and formaldehyde in order to give the acid of formula (Ia).

10. Process as claimed in any one of Claims 1 to 8, characterised in that when applied to the preparation of the acrylic acid of formula 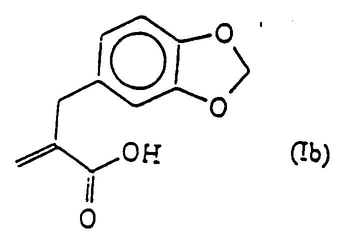 it consists of
- condensing a carbonyl compound of formula (XI), piperonal, in a Knoevenagel reaction with a malonate of formula (X) in the presence of a base such as piperidine and an organic acid such as acetic acid in an organic solvent such as toluene,
- subjecting the esters thus formed to a catalytic hydrogenation,
- saponification of the diesters by an alkaline aqueous solution such as a solution of soda,
- liberation by acidification of the diacid of formula (XIIIb) 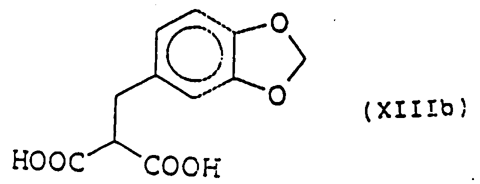 then extracting it with an organic solvent such as ethyl acetate,
- and subjecting the solution to the action of a base such as diethylamine and formaldehyde in order to give the acid of formula (Ib).

11. Process as claimed in any one of Claims 1 to 8, characterised in that when applied to the preparation of the acrylic acid of formula 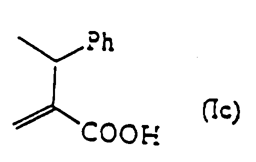 it consists of
- causing a halide of formula (IX), such as a chlorine derivative in which R₁ is a phenyl group, R₂ is a methyl group, Y designates chlorine and n = 0, to react with a malonic ester of formula (X) in the presence of an alcoholic solution of an alkaline metal such as a solution of sodium in ethanol or in methanol,
- saponification of the diester thus obtained with an alkaline aqueous solution such as an aqueous solution of soda,
- liberation by acidification of the diacid of formula (XIIIc) 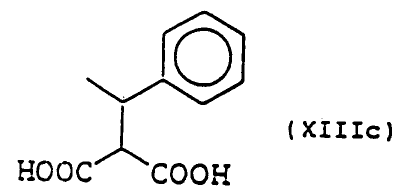 and extracting it with an organic solvent such as ethyl acetate,
- and subjecting the solution to the action of a base such as diethylamine and formaldehyde in order to give the acid of formula (Ic).

12. Process for the synthesis of N-(mercaptoacyl)amino acids of formula (II) 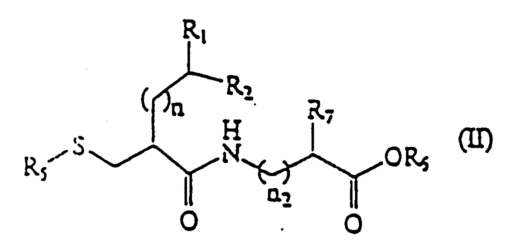 in which
R₁ and R₂ have the same meaning as in formula (I) according to Claim 1,
n varies from 0 to 10,
R₅ represents a hydrogen atom, a linear or branched aliphatic acyl radical or an aromatic acyl radical,
R₆ represents a hydrogen atom, a C₁-C₆ alkyl radical, a phenyl radical or a phenyl(C₁-C₆ alkylene) group,
n₂ varies from 0 to 10,
R₇ represents a hydrogen atom; a C₁-C₆ alkyl group; a C₁-C₆ hydroxyalkylene group; a phenyl group; a phenyl(C₁-C₆ alkylene) group; a hydroxyphenyl(C₁-C₆ alkylene) group; a C₁-C₆ aminoalkylene group; a guanidino(C₁-C₆ alkylene) group; a C₁-C₆ mercaptoalkylene group; a (C₁-C₆ alkyl)C₁-C₆ thioalkylene group; an imidazolyl(C₁-C₆ alkylene) group; an indolyl(C₁-C₆ alkylene) group; a carbamyl(C₁-C₆ alkylene) group; a carboxy(C₁-C₆ alkylene) group,
in which process
- an acid of formula (I) is prepared in accordance with any one of Claims 1 to 11,
- the acid of formula (I) thus obtained is subjected to a Michael addition with a sulphur derivative of formula R₅-SH in order to form the acid of formula (XIV) 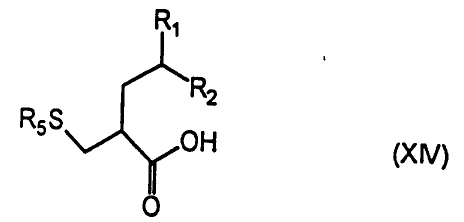
where R₁, R₂ and R₅ have the same meanings as were given in formula (II),
- the acid of formula (XIV) is possibly divided in two,
- the acid of formula (XIV) in racemic or optically pure form is coupled with an amino ester of formula (XV) 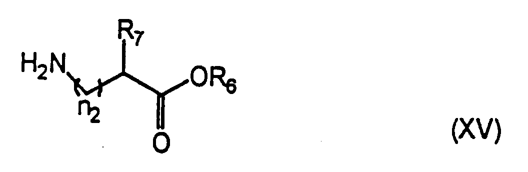
where R₆, R₇ and n₂ have the meanings which were given in formula (II); in order to form the derivatives of formula (II).

13. Process as claimed in Claim 12, characterised in that the acrylic acid of formula (Ia) according to Claim 9 is prepared for the synthesis of the derivatives of formula (II).

14. Process as claimed in Claim 13, characterised in that the acrylic acid of formula (Ia) according to Claim 9 is prepared for the synthesis of benzyl N-(RS)-[2-acetylthiomethyl-1-oxo-3-phenylpropyl]-glycinate.

15. Process as claimed in Claim 13, characterised in that the acrylic acid of formula (Ia) according to Claim 9 is prepared for the synthesis of benzyl N-(S)-[2-acetylthiomethyl-1-oxo-3-phenylpropyl]-glycinate.

16. Process as claimed in Claim 13, characterised in that the acrylic acid of formula (Ia) according to Claim 9 is prepared for the synthesis of benzyl N-(R)-[2-acetylthiomethyl-1-oxo-3-phenylpropyl]-glycinate.

17. Process as claimed in Claim 12, characterised in that the acrylic acid of formula (Ib) according to Claim 10 is prepared for the synthesis of the derivatives of formula (II).

18. Process as claimed in Claim 17, characterised in that the acrylic acid of formula (Ib) according to Claim 10 is prepared for the synthesis of benzyl N-(S)-[2-acetylthiomethyl-1-oxo-3(3,4-methylenedioxyphenyl)propyl]-alaninate.

19. Process as claimed in Claim 12, characterised in that the acrylic acid (Ic) according to Claim 11 is prepared for the synthesis of the derivatives of formula (II).

20. Process as claimed in Claim 19, characterised in that the acrylic acid (Ic) according to Claim 11 is prepared for the synthesis of N-[(2S,3R)-2-benzoylthiomethyl-1-oxo-3-phenylbutyl]-(S)-alanine.

## Patentansprüche

1. Verfahren zur Herstellung α-substituierter Acrylsäuren mit der Formel (I) 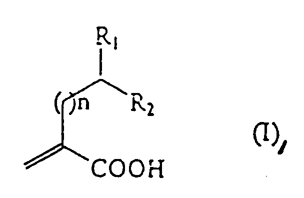 in welcher
R₁ ein Wasserstoffatom, eine Alkylgruppe, eine Cycloalkylgruppe, eine gegebenenfalls durch ein Halogenatom, eine Trifluormethylgruppe, Nitrogruppe, Cyanogruppe, Aminogruppe, Dimethylaminogruppe, Hydroxygruppe, C₁- bis C₆-Alkoxygruppe, Phenoxygruppe, Benzyloxygruppe, Methylthiogruppe, Phenylgruppe, C₁- bis C₆-Alkylgruppe und (C₁- bis C₆-Alkylen)phenylgruppe mono- oder polysubstituierte Phenylgruppe, eine α- und β-Naphthylgruppe und eine Gruppierung 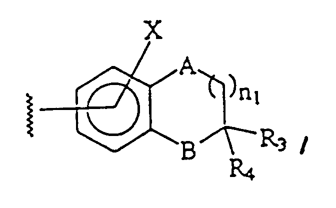 bedeutet, worin
A eine Methylengruppe, ein Sauerstoffatom, ein Schwefel- oder Stickstoffatom,
B eine/s der für die Definition von A genannten Gruppen oder Atome, wobei n₁ gleich 0 oder 1 ist,
X ein Wasserstoffatom, ein Halogenatom, eine Hydroxy-, C₁- bis C₆-Alkylgruppe oder Trifluormethylgruppe,
R₃ ein Wasserstoffatom, eine Phenylgruppe, eine C₁- bis C₆-Alkylgruppe, ein Halogenatom oder eine Trifluormethylgruppe,
R₄ ebenfalls ein Wasserstoffatom oder eine der für die Definition von R₃ genannten Gruppen und
R₂ ein Wasserstoffatom oder eine der für die Definition von R₁ genannten Gruppen bedeutet, wobei n von 0 bis 10 variiert, **dadurch gekennzeichnet**, **daß** nacheinander
a) Malonsäurealkylester mit der Formel (VIII) 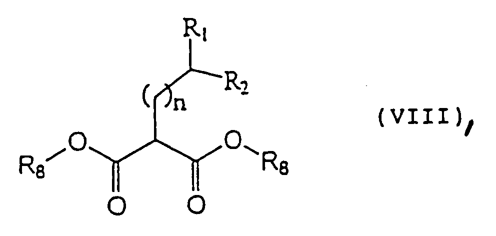 in welcher n, R₁ und R₂ dieselbe Bedeutung wie in Formel (I) haben und R₈ eine 1 bis 4 Kohlenstoffatome enthaltende Alkylkette bedeutet,
hergestellt werden, wobei die Malonsäurealkylester
- entweder, indem ein Halogenid mit der Formel (IX) 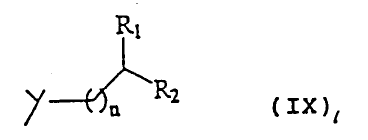 in welcher n, R₁ und R₂ dieselbe Bedeutung wie in Formel (I) haben und Y ein Halogenatom bedeutet, mit einem Malonsäureester der Formel (X) 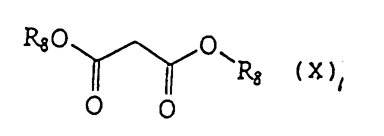 in welcher R₈ obengenannte Bedeutung hat, in Gegenwart einer alkoholischen Lösung eines Alkalimetalls umgesetzt wird oder
- durch Knoevenagel-Kondensation einer Carbonylverbindung mit der Formel (XI) oder (XI') 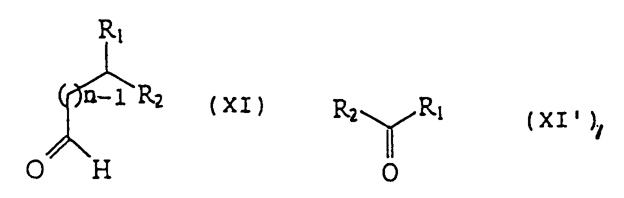 in welchen R₁ und R₂ dieselbe Bedeutung wie in Formel (I) haben und n von 1 bis 10 variiert, mit einem Malonsäureester der Formel (X) in Gegenwart einer Base und einer Carbonsäure, um Ester mit der Formel (XII) oder (XII') 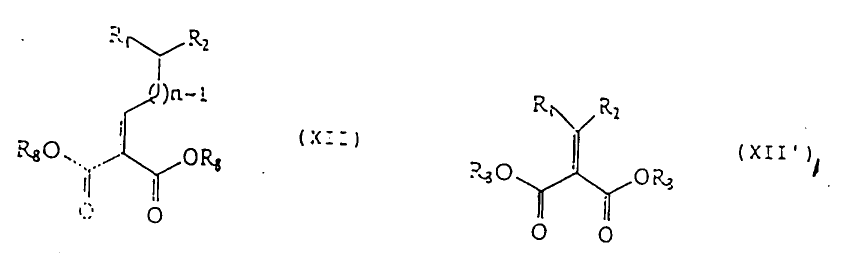 zu ergeben, in welchen R₁, R₂ und R₈ obengenannte Bedeutungen haben und n von 1 bis 10 variiert, wobei die Ester mit der Formel (XII) oder (XII') anschließend einer katalytischen Hydrierung unterworfen werden, um Ester mit der Formel (VIII) zu bilden, erhalten werden,
b) die Ester mit der Formel (VIII) in Gegenwart einer wäßrigen basischen Lösung verseift werden, um Disäuren mit der Formel (XIII) 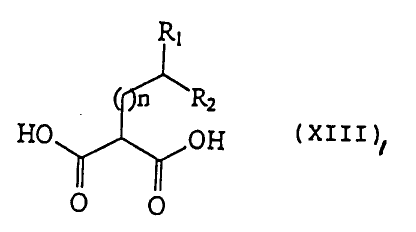 in welcher n, R₁ und R₂ dieselbe Bedeutung wie in Formel (I) haben, zu bilden, und
c) die Disäuren mit der Formel (XIII) einer Mannich-Reaktion mit einer organischen Base und Formaldehyd unterworfen werden, um die Acrylsäuren mit der Formel (I) zu bilden.

2. Verfahren nach Anspruch 1, **dadurch gekennzeichnet**, **daß** das Halogenid mit der Formel (IX) ein Chlor-, Brom- oder Iodderivat ist.

3. Verfahren nach Anspruch 1 oder 2, **dadurch gekennzeichne**t, **daß** die alkoholische Lösung eines Alkalimetalls eine Lösung von Natrium in Ethanol oder Methanol ist.

4. Verfahren nach einem der Ansprüche 1 bis 3, **dadurch gekennzeichnet**, **daß** die für die Knoevenagel-Reaktion eingesetzte Base Piperidin ist.

5. Verfahren nach einem der Ansprüche 1 bis 4, **dadurch gekennzeichnet**, **daß** die für die Knoevenagel-Reaktion eingesetzte Carbonsäure aus Essig- und Benzoesäure ausgewählt wird.

6. Verfahren nach einem der Ansprüche 1 bis 5, **dadurch gekennzeichne**t, **daß** man die katalytische Hydrierung der Verbindungen mit den Formeln (XII) und (XII') durchführt, indem Palladium auf Aktivkohle als Katalysator verwendet wird.

7. Verfahren nach einem der Ansprüche 1 bis 6, **dadurch gekennzeichnet**, **daß** die für die Verseifung der Diester mit der Formel (VIII) verwendete wäßrige basische Lösung Natronlauge ist.

8. Verfahren nach einem der Ansprüche 1 bis 7, **dadurch gekennzeichnet**, **daß** die für die Mannich-Reaktion verwendete Base aus Diethylamin, Dimethylamin und Piperidin ausgewählt wird.

9. Verfahren nach einem der Ansprüche 1 bis 8, **dadurch gekennzeichnet**, **daß** es darin besteht, wenn es für die Herstellung einer Acrylsäure der Formel 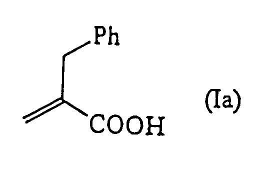 angewendet wird, eine Knoevenagel-Kondensation einer Carbonylverbindung der Formel (XI), Benzaldehyd, mit einem Malonat der Formel (X) in Gegenwart einer Base wie Piperidin und einer Carbonsäure wie Essigsäure in einem Lösungsmittel wie Toluol durchzuführen,
- die erhaltenen Ester einer katalytischen Hydrierung zu unterwerfen,
- die Diester durch eine wäßrige alkalische Lösung wie Natronlauge zu verseifen,
- die Disäure mit der Formel (XIIIa) 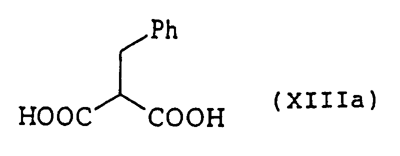 durch Ansäuern freizusetzen und durch ein organisches Lösungsmittel wie Ethylacetat zu extrahieren und
- die Lösung der Einwirkung einer Base wie Diethylamin und von Formaldehyd zu unterwerfen, um die Säure mit der Formel (Ia) zu bilden.

10. Verfahren nach einem der Ansprüche 1 bis 8, **dadurch gekennzeichnet**, **daß** es darin besteht, wenn es angewendet wird für die Herstellung von Acrylsäure der Formel 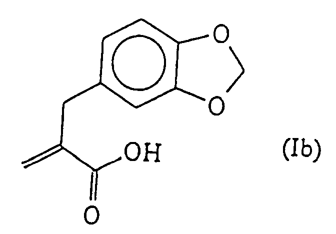
- eine Carbonylverbindung mit der Formel (XI), Piperonal, entsprechend einer Knoevenagel-Reaktion mit einem Malonat der Formel (X) in Gegenwart einer Base wie Piperidin und einer organischen Säure wie Essigsäure in einem organischen Lösungsmittel wie Toluol zu kondensieren,
- die gebildeten Ester einer katalytischen Hydrierung zu unterwerfen,
- die Diester durch eine wäßrige alkalische Lösung wie Natronlauge zu verseifen,
- die Disäure mit der Formel (XIIIb) 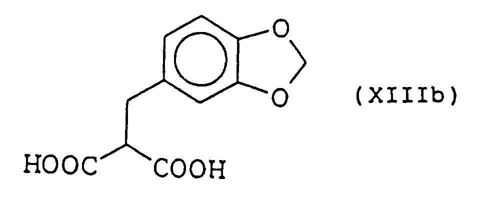 durch Ansäuern freizusetzen und anschließend durch ein organisches Lösungsmittel wie Ethylacetat zu extrahieren und
- die Lösung der Einwirkung einer Base wie Diethylamin und von Formaldehyd zu unterwerfen, um die Säure mit der Formel (Ib) zu bilden.

11. Verfahren nach einem der Ansprüche 1 bis 8, **dadurch gekennzeichnet**, **daß** es darin besteht, wenn es angewendet wird für die Herstellung von Acrylsäure der Formel 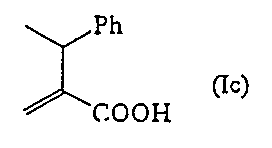
- ein Halogenid mit der Formel (IX) wie ein Chlorderivat, in welchem R₁ eine Phenylgruppe, R₂ eine Methylgruppe und Y Chlor bedeutet und n = 0 ist, mit einem Malonsäureester der Formel (X) in Gegenwart einer alkoholischen Lösung eines Alkalimetalls wie einer Lösung von Natrium in Ethanol oder Methanol umzusetzen,
- den erhaltenen Diester durch eine wäßrige alkalische Lösung wie Natronlauge zu verseifen,
- die Disäure mit der Formel (XIIIc) 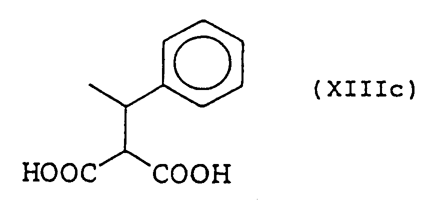 durch Ansäuern freizusetzen und durch ein organisches Lösungsmittel wie Ethylacetat zu extrahieren und
- die Lösung der Einwirkung einer Base wie Diethylamin und von Formaldehyd zu unterwerfen, um die Säure mit der Formel (Ic) zu bilden.

12. Verfahren zur Herstellung von N-(Mercaptoacyl)-aminosäuren mit der Formel (II) 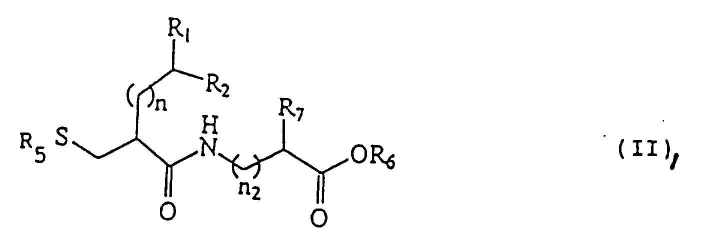 gemäß Anspruch 1, in welcher
R₁ und R₂ dieselbe Bedeutung wie in Formel (I) haben,,
n von 0 bis 10 variiert,
R₅ ein Wasserstoffatom, einen geradkettigen bzw. verzweigten aliphatischen oder einen aromatischen Acylrest bedeutet,
R₆ ein Wasserstoffatom, einen C₁- bis C₆-Alkylrest, einen Phenylrest oder eine (C₁- bis C₆-Alkylen)phenylgruppe bedeutet,
n₂ von 0 bis 10 variiert und
R₇ ein Wasserstoffatom, eine C₁- bis C₆-Alkylgruppe, C₁- bis C₆-Hydroxyalkylengruppe, Phenylgruppe, (C₁- bis C₆-Alkylen)-phenylgruppe, (C₁- bis C₆-Alkylen)-hydroxyphenylgruppe, C₁- bis C₆-Aminoalkylengruppe, (C₁- bis C₆-Alkylen)-guanidinogruppe, C₁- bis C₆-Mercaptoalkylengruppe, (C₁- bis C₆-Alkyl)-C₁- bis C₆-thioalkylengruppe, (C₁- bis C₆-Alkylen)-imidazolylgruppe, (C₁- bis C₆-Alkylen)-indolylgruppe, (C₁- bis C₆-Alkylen)-carbamylgruppe und (C₁- bis C₆-Alkylen)-carboxygruppe bedeutet, wobei im Verfahren
- eine Säure mit der Formel (I) gemäß einem der Ansprüche 1 bis 11 hergestellt wird,
- die so erhaltene Säure mit der Formel (I) einer Michael-Addition mit einem Schwefelderivat der Formel R₅-SH unterworfen wird, um eine Säure der Formel (XIV) 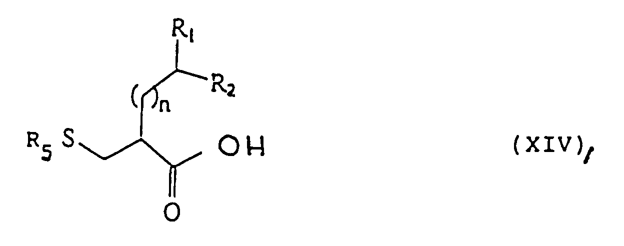 zu bilden, in welcher R₁, R₂ und R₅ die für Formel (II) angegebenen Bedeutungen haben,
- die Säure mit der Formel (XIV) gegebenenfalls gespalten wird und
- die Säure mit der Formel (XIV), die in Form eines razemischen Gemischs oder optisch rein vorliegt, mit einem Aminoester der Formel (XV) 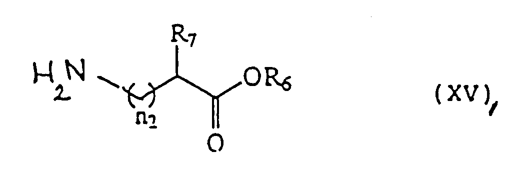 in welcher R₆, R₇ und n₂ die für Formel (II) angegebenen Bedeutungen haben, verbunden wird, um Derivate der Formel (II) zu bilden.

13. Verfahren nach Anspruch 12, **dadurch gekennzeichnet**, **daß** zur Herstellung der Derivate der Formel (II) Acrylsäure der Formel (Ia) gemäß Anspruch 9 hergestellt wird.

14. Verfahren nach Anspruch 13, **dadurch gekennzeichnet**, **daß** zur Herstellung von N-(RS)-[2-acetylthiomethyl-1-oxo-3-phenylpropyl]-benzylglycinat Acrylsäure der Formel (Ia) gemäß Anspruch 9 hergestellt wird.

15. Verfahren nach Anspruch 13, **dadurch gekennzeichnet**, **daß** zur Herstellung von N-(S)-[2-acetylthiomethyl-1-oxo-3-phenylpropyl]-benzylglycinat Acrylsäure der Formel (Ia) gemäß Anspruch 9 hergestellt wird.

16. Verfahren nach Anspruch 13, **dadurch gekennzeichnet**, **daß** zur Herstellung von N-(R)-[2-acetylthiomethyl-1-oxo-3-phenylpropyl]-benzylglycinat Acrylsäure der Formel (Ia) gemäß Anspruch 9 hergestellt wird.

17. Verfahren nach Anspruch 12, **dadurch gekennzeichnet**, **daß** zur Herstellung der Derivate der Formel (II) Acrylsäure der Formel (Ib) gemäß Anspruch 10 hergestellt wird.

18. Verfahren nach Anspruch 17, **dadurch gekennzeichnet**, **daß** zur Herstellung von N-(S)-[2-acetylthiomethyl-1-oxo-3-(3,4-methylendioxyphenyl)propyl]-(S)-benzylalaninat Acrylsäure der Formel (Ib) gemäß Anspruch 10 hergestellt wird.

19. Verfahren nach Anspruch 12, **dadurch gekennzeichnet**, **daß** zur Herstellung der Derivate der Formel (II) Acrylsäure gemäß Anspruch 11 hergestellt wird.

20. Verfahren nach Anspruch 19, **dadurch gekennzeichnet**, **daß** zur Herstellung von N-[(2S, 3R)-2-benzoylthiomethyl-1-oxo-3-phenylbutyl]-(S)-alanin Acrylsäure (Ic) gemäß Anspruch 11 hergestellt wird.
